# EUROPEAN PATENT APPLICATION

(11) **EP 2 778 165 A1**
(43) Date of publication of application: **17.09.2014**
(21) Application number: 14159607.2
(22) Date of filing: 13.03.2014
(51) Int. Cl.: C07D 405/12

(54) **Synthesis of a serotonin reuptake inhibitor**

(30) Priority: 15.03.2013 IT MI20130392
(71) Applicant: Dipharma Francis S.r.l., 20021 Baranzate (MI) (IT)
(72) Inventor: Taddei, Maurizio, 20021 BARANZATE (MI) (IT); Giannotti, Luca, 20021 BARANZATE (MI) (IT); Attolino, Emanuele, 20021 BARANZATE (MI) (IT); Allegrini, Pietro, 20021 BARANZATE (MI) (IT)
(74) Representative: Bertuccio, Silvia

(57) **Abstract**

Process for the preparation of the piperazinyl derivative vilazodone, having activity as a serotonin reuptake inhibitor and used in therapy for treating serious symptoms of depression in adults, and its synthetic intermediates.

## Description

### TECHNICAL FIELD OF INVENTION

The present invention relates to a novel process for the preparation of vilazodone, and its intermediates, having activity as a serotonin reuptake inhibitor and used in therapy for treating serious symptoms of depression.

### PRIOR ART

5-(4-[4-(5-cyano-1*H*-indol-3-yl)butyl]piperazin-1-yl)benzofuran-2-carboxamide of formula **(I),** also known as vilazodone, is a serotonin reuptake inhibitor, which acts as a partial 5-HT1A receptor agonist. Said compound is marketed as hydrochloride salt and used to treat serious symptoms of depression in adults, at the doses of 10, 20 and 40 mg.

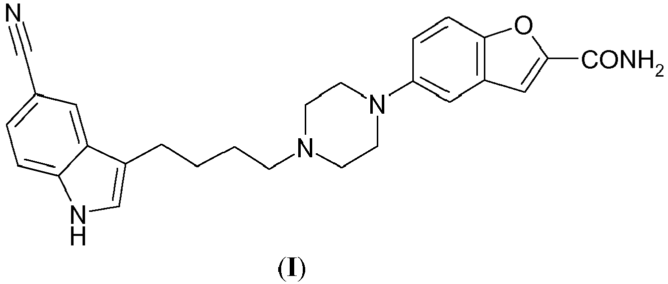

Vilazodone is known from US 5,532,241, which describes its synthesis (Scheme 1) by means of a process involving alkylation of the piperazine derivative of formula **A** with an alkylating agent of formula **B** to obtain the piperazine compound of formula **C,** which, after conversion of the carboxyl functional group to primary amide, affords vilazodone.

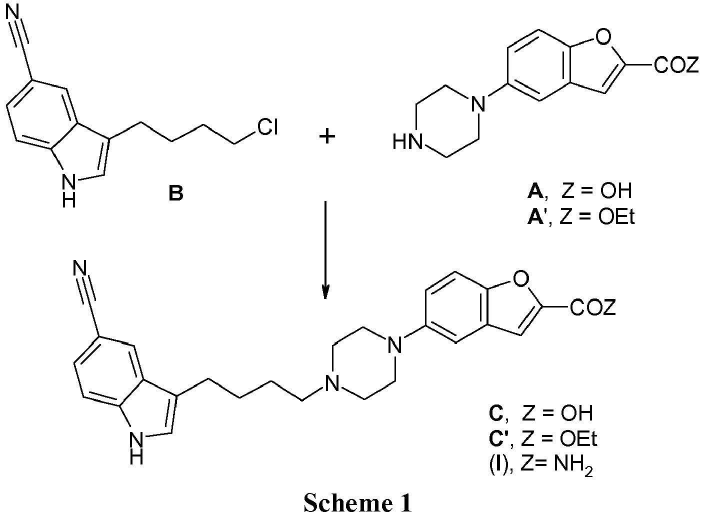

In J. Med. Chem. 2004, 47, 4684-4692, however, the same researchers as in US 5,532,241 propose the alkylation of piperazine of formula **A',** which provides the ester derivative **C',** giving rise to vilazodone after conversion of the ester functional group to amide. As regards the synthesis of the piperazines of formulas **A** and **A',** besides the one reported in the said J. Med. Chem. 2004, 47, 4684-4692, numerous fairly efficient preparation examples are described in the literature. However, the preparation of an alkylating agent like the one used in formula **B** is rather complex and expensive in industrial terms.

For example in J. Med. Chem. 2004, 47, 4684-4692, **B** is obtained by reduction of the carbonyl derivative **D** in accordance with the synthesis scheme shown below (Scheme 2):

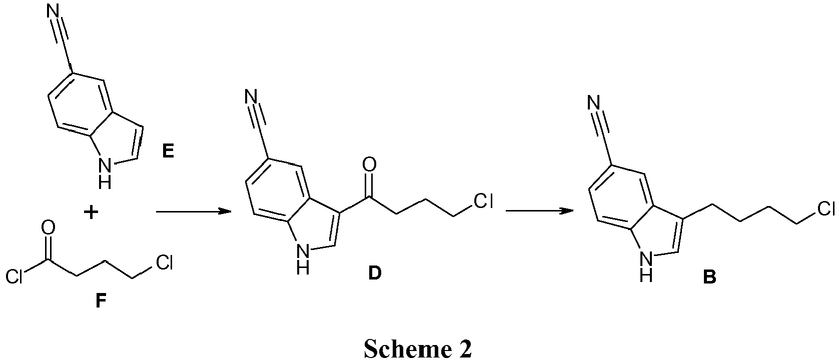

Said compound of formula **D** is prepared in its turn by Friedel Crafts reaction of 5-cyanoindole of formula **E** with chlorobutyroyl chloride of formula **F,** both of which are commercially available.

The carbonyl derivative **D** is reduced with the expensive bis-(2-methoxyethoxy)aluminium hydride, also known as Vitride/Red Al, with a yield of 26% after chromatographic purification.

However, an article published very recently in Org. Process. Res. Dev. 2012, 16, 1552-1557 states that to eliminate this problem, the authors have tested a series of reducing systems and have identified the system NaBH₄/trifluoroacetic acid (TFA) in dichloromethane as the best to provide product B with a yield of 95%.

If the said reduction process is carefully analysed, however, it will be seen that it is very expensive, precisely because of the use of TFA as co-solvent and a chlorinated solvent as reaction solvent. No less important is the fact that the inventors of the present invention have established that in safety terms, under the conditions reported above, said reaction produces a great deal of energy. Thus when the addition of NaBH₄ to the reaction mixture was tested in the laboratory, the reaction proved highly exothermic and violent, and consequently very dangerous for the personnel involved. Said method is therefore unsuitable for preparation on an industrial scale.

There is consequently a need for an alternative preparation method of preparing vilazodone of formula **(I),** which is simpler and more advantageous. Said novel method should in particular involve the use of intermediates, which are more easily synthesisable and purifiable on an industrial scale, and obtainable by cheap processes, which are safe for humans and the environment, preferably catalytic, in order to obtain the vilazodone of formula **(I)** with high yields and efficiency.

### SUMMARY OF THE INVENTION

It has now surprisingly been found that a compound of formula **(II),** 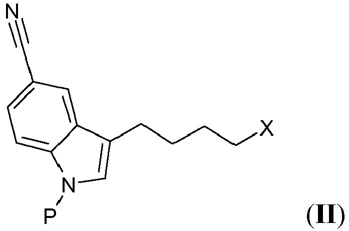 wherein X is an -OH group or a leaving group and P is H or a protecting group of the indole nitrogen atom, can be advantageously prepared by a process comprising the reduction of a compound of formula **(III)** or **(IV)** 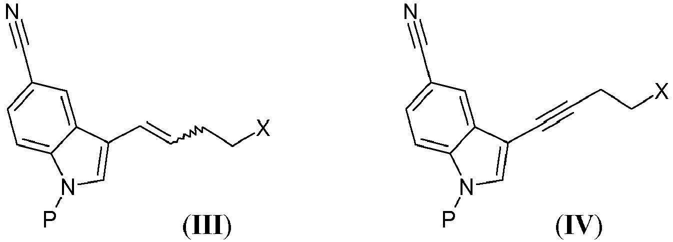 wherein X and P are as defined above and the double bond -C=C- on the aliphatic chain can have (E) or (Z) stereochemistry or a mixture thereof, and, if desired, the conversion of a compound of formula **(II)** thus obtained to another compound of formula **(II).**

Said compounds of formulas **(III)** and **(IV)** are novel, and also are a subject of the invention.

The reduction reaction of the process according to the invention is particularly advantageous because it is controllable, safe and cheap, the reaction conditions are particularly mild, and the desired compound of formula **(II)** is obtained with high yields and chemical purity. Vilazodone of formula **(I),** or a salt thereof, obtained with the intermediates and process to which the present invention relates, is therefore produced with high yields and chemical purity.

### DETAILED DESCRIPTION OF THE INVENTION

The subject of the present invention is a process for the preparation of a compound of formula **(II)** 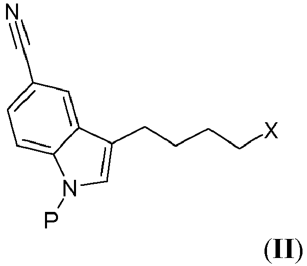 wherein X is an -OH group or a leaving group and P is H or a protecting group of the indole nitrogen atom, comprising the reduction of a compound of formula **(III)** or a compound of formula **(IV)** 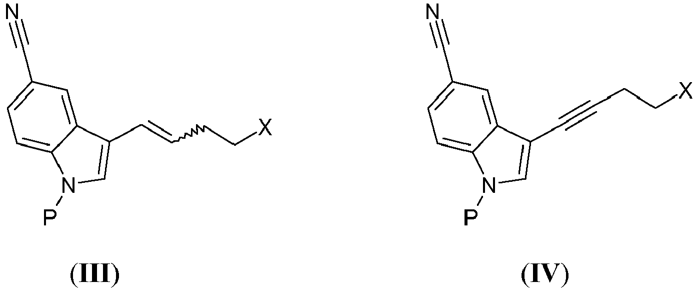 wherein X and P are as defined above and, if desired, conversion of a compound of formula **(II)** to another compound of formula **(II).**

In a compound of formula **(III),** the double bond -C=C- on the aliphatic chain can have either (E) or (Z) stereochemistry, or a mixture thereof.

A leaving group is, for example, a halogen atom, preferably chlorine or iodine, or an OSO₂R group, wherein R is an optionally substituted straight or branched C₁-C₆ alkyl group, an optionally substituted aryl group or an optionally substituted heteroaryl group. A C₁-C₆ alkyl group is preferably a C₁-C₄ alkyl group, such as methyl, ethyl or butyl, which can be substituted by one or more substituents selected independently from halogen and phenyl, which in turn is optionally substituted by halogen or nitro, typically trifluoromethyl, nonafluorobutyl, benzyl, p-bromobenzyl and p-nitrobenzyl. An aryl group can be, for example, phenyl optionally substituted by a C₁-C₄ alkyl group, and is preferably p-tolyl. A heteroaryl group can be, for example, N-imidazole. Said leaving group is preferably methanesulphonyl.

More preferably, X is an -OH or methanesulphonyl group.

A protecting group of the indole nitrogen atom is an amino protecting group well known to the skilled person. For example, the indole nitrogen atom can be protected as a carbamate, amide or sulphonamide functional group.

A protecting group P is, for example, an acyl group, typically an acetyl, tert-butyloxycarbonyl (Boc) or benzyloxycarbonyl (Cbz) group or an OSO₂P' group, wherein P' is for example, methyl, ethyl, phenyl, benzyl, p-tolyl or trifluoromethyl.

P is preferably a p-toluensulphonyl group (tosyl) or Boc.

A compound of formula **(III)** or formula **(IV)** can be reduced, for example, by catalytic hydrogenation in the presence of a homogenous or heterogeneous metal catalyst, such as one based on Pd, Pt, Ni, Rh or Ru, preferably based on Pd. When the metal catalyst is heterogeneous, it is preferably supported on an inert support such as charcoal, barium hydroxide, alumina or calcium carbonate, preferably charcoal.

The metal catalyst is preferably Pd(OH)₂/C or Pd/C.

The concentration of metal on the support can range between about 1 and 30%, preferably between about 5 and 20%.

The hydrogen pressure used can range between about 1 atm and 10 atm, but the reaction is preferably performed at atmospheric pressure.

The molar quantity of catalyst used, with respect to the compound of formula **(II),** is between about 0.1 and 10% w/w, preferably between about 0.5 and 5% w/w.

The reaction can be performed in the presence of an organic solvent selected, for example, from a polar aprotic solvent, typically dimethylformamide, dimethylacetamide, acetonitrile or dimethyl sulphoxide; a cyclic or acyclic ether, typically tetrahydrofuran, dioxane or methyl-tertbutyl ether; a chlorinated solvent, typically dichloromethane; an apolar aprotic solvent, typically toluene or hexane; a polar protic solvent, such as a straight or branched C₁-C₆ alkanol, in particular methanol, ethanol, isopropanol or butanol, or water; an ester, such as ethyl acetate, isopropyl acetate or butyl acetate; a straight or branched C₃-C₇ ketone, such as acetone, methylethyl ketone or methyl isobutyl ketone; a carboxylic acid, such as acetic acid or propionic acid; or mixtures of two or more of said solvents, preferably 2 or 3 thereof. Alternatively, the reaction can be performed in a solution of a mineral acid, such as hydrochloric acid or sulphuric acid, or a mixture thereof with one, two or three of the organic solvents listed above.

The reaction can preferably be performed in an alkanol, as defined above, or a mixture of two to four, typically two or three, alkanols, or a mixture thereof with water, or in an acetonitrile/water mixture; more preferably the reduction is performed in methanol or tetrahydrofuran (THF).

Said reduction reaction of a compound of formula **(III)** or **(IV),** as defined above, can be performed at a temperature ranging between about 0°C and the reflux temperature of the solvent, preferably between about 0°C and ambient temperature.

The reduction reaction of a compound of formula **(III)** or formula **(IV)** can also be performed by hydrogen transfer reaction, using a homogenous or heterogeneous metal catalyst, for example as defined above and in the same molar amount, and a hydrogen donor.

A hydrogen donor is selected, for example, from the group containing cyclohexene, cyclohexadiene, methylcyclohexene, limonene, dipentene, mentene, hydrazine, phosphonic acid or derivatives thereof, such as sodium hypophosphite, indoline, ascorbic acid, formic acid or sodium or ammonium salts thereof, and a secondary alcohol, such as isopropanol; preferably a hydrogen donor is ammonium formate.

The molar ratio between the hydrogen donor and the compound of formula **(III)** or formula **(IV)** can range between about 1.5 and 50, preferably between about 1.5 and 10.

The hydrogen transfer reduction reaction can be performed in the presence of an organic solvent selected, for example, from one of the above-mentioned solvents.

The conversion of a compound of formula **(II)** to another compound of formula **(II)** can be effected by known methods.

For example a compound of formula **(II),** wherein X is -OH, can be converted to a compound of formula **(II)** wherein X is -OSO₂CH₃, by reaction with methanesulphonyl chloride in the presence of an organic base, such as a tertiary amine, in particular triethylamine or diisopropylethylamine, or an inorganic base such as potassium carbonate.

According to an aspect of the process of the invention in a compound of formula **(IV)** X and P are as defined above, and in a compound of formula **(III)** X and P, being as defined above, X is other than O-CH₂-phenyl, and P is other than tosyl.

A further subject of the present invention is the use as intermediate of a compound of formula **(II),** wherein X and P are as defined above, and in particular X is a leaving group, obtained according to the process of the invention, to prepare a compound of formula **(I),** namely vilazodone.

In particular, a compound of formula **(I),** or a salt thereof, can be obtained by
a process comprising alkylation of a compound of formula **(VIII),** or a salt thereof 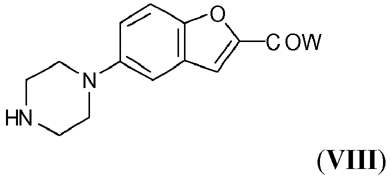 wherein W is an -OH group or an -OR₁ group, wherein R₁ is a straight or branched C₁-C₆ alkyl group, or -NH₂, with a thus obtained compound of formula **(II),** wherein X is a leaving group as defined above and P is H or a protecting group as defined above, to obtain a compound of formula **(IX)** or a salt thereof 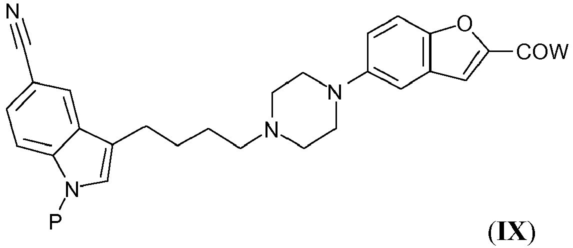 wherein W and P are as defined above and, if applicable, the conversion of a compound of formula **(IX)** to another compound of formula **(IX),** and/or the removal of the protecting group to obtain a compound of formula **(I)** or a salt thereof.

The alkylation reaction between a compound of formula **(II)** and a compound of formula **(VIII)** to obtain a compound of formula **(IX),** and the subsequent conversion to vilazodone of formula **(I),** can be obtained according to known methods, for example as disclosed in US 5,532,241, Org. Process Res. Dev. 2012, 16, 1552-1557 or J. Med. Chem. 2004, 47, 4684-4692.

The conversion of a compound of formula **(IX)** to another compound of formula **(IX)** can be effected by methods known in the prior art, for example as described in US 5,532,241 or Org. Process Res. Dev. 2012, 16, 1552-1557 or J. Med. Chem. 2004, 47, 4684-4692.

A salt of a compound of formula **(VIII)** or **(IX)** or **(I)** according to the present invention is preferably a pharmaceutically acceptable salt thereof.

A compound of formula **(III)** can be prepared by reacting a compound of formula **(V)** 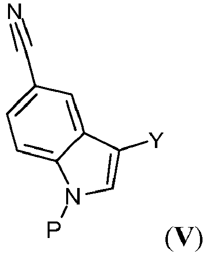 wherein Y is a halogen, preferably bromine or iodine, or an OSO₂ R group, wherein R, being as defined above, is for example, methyl, ethyl, phenyl, benzyl, p-tolyl, p-bromobenzyl, p-nitrobenzyl, trifluoromethyl, nonafluorobutyl or N-imidazole, preferably methyl; and P is as defined above, with a compound of formula **(VI)** 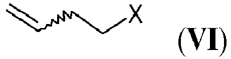 wherein X is as defined above, under the Heck reaction conditions, which are well known to the skilled person.

For example, the Heck reaction between a compound of formula **(V)** and a compound of formula **(VI)** can be effected in the presence of a catalyst based on Pd(0), preformed or generated *in situ* from a Pd(II) salt, in particular PdCl₂ or Pd(OAc)₂, if desired in the presence of a binder such as triphenylphosphine, and an organic or inorganic base, in particular a secondary or tertiary amine, optionally in the presence of a solvent such as a dipolar aprotic solvent, typically dimethylformamide, dimethylacetamide, acetonitrile, dimethyl sulphoxide.

A compound of formula **(IV)** can be prepared by reacting a compound of formula **(V),** as defined above, with a compound of formula **(VII),** 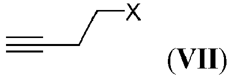 wherein X is as defined above, under the Sonogashira reaction conditions, which are well known to the skilled person.

For example, the Sonogashira reaction between a compound of formula **(V)** and a compound of formula **(VII)** can be effected in the presence of a catalyst based on Pd(0), preformed or generated in situ from a Pd(II) salt, in particular PdCl₂ or Pd(OAc)₂, if desired in the presence of a binder such as triphenylphosphine, if desired in the presence of a co-catalyst based on Cu(I), such as Cul, and an organic or inorganic base, in particular a secondary or tertiary amine, preferably triethylamine (TEA), optionally in the presence of a solvent such as a dipolar aprotic solvent, typically dimethylformamide (DMF), dimethylacetamide (DMA), acetonitrile or dimethyl sulphoxide or in a cyclic ether such as tetrahydrofuran. The Sonogashira reaction is preferably carried out in dimethylformamide (DMF), dimethylacetamide (DMA) or in tetrahydrofuran (THF).

The use of a compound of formula **(III)** and formula **(IV)** in preparing a compound of formula **(IX)** or **(I)** as defined above, or a salt thereof, is novel, constituting a further subject of the present invention.
A preferred aspect of the invention is a compound of formula **(III)** or **(IV),** wherein X is -OH or a leaving group, and P is H or a protecting group of the indole nitrogen and, in a compound of formula **(III),** the double bond -C=C- on the aliphatic chain has either (E) or (Z) stereochemistry, or a mixture thereof; and wherein when in a compound of formula **(III)** P is tosyl, X is other than OCH₂Ph.

The compounds of formula **(VI)** and formula **(VII)** are commercially available.

The compound of formula **(V)** can be prepared by well-known methods reported in literature, for example, as disclosed in US 7,105,516.

The following examples further illustrate the invention.

### Example 1: Synthesis of 3-iodo-1H-indole-5-carbonitrile (V)

1H-indole-5-carbonitrile (5 g, 35.2 mmol), KOH (7.90 g, 141 mmol) and I₂ (8.90 g, 35.2 mmol) are suspended in 25 mL of DMF under inert atmosphere. The reaction is maintained under stirring in the dark for 30 min. at 10°C, and then treated with an 0.1M solution of Na₂S₂O₃ (150 mL). The resulting suspension is maintained under stirring for 30 min, then filtered, and the resulting solid is washed with water and dried at 50°C under vacuum to constant weight. Product **(V)** (9.0 g) is obtained as a white solid with a yield of 95%.

**¹H-NMR (400 MHz, CDCl₃), δ:** 8.78 (1H, bs); 7.80 (1H, s); 7.46-7.40 (3H, m).

### Example 2: Synthesis of 3-iodo-1-tosyl-1H-indole-5-carbonitrile (V)

3-iodo-1H-indole-5-carbonitrile (V), obtained as described in Example 1 (8.7 g, 32.5 mmol), NaOH (2.1 g, 52.5 mmol), triethylbenzylammonium chloride (0.7 g, 3.1 mmol) and tosyl chloride (6.7g, 35.1 mmol) are suspended in CH₂Cl₂ (260 mL) under inert atmosphere, and the reaction mixture is maintained under stirring at ambient temperature overnight. The mixture is treated with water and the phases are separated. The organic phase is further washed with water and a saturated solution of NaCl, then dried on Na₂SO₄, filtered and concentrated at low pressure. Product **(V)** is thus obtained as a solid (12.5 g) with a yield of 91%.

**¹H-NMR (400 MHz, CDCl₃), δ:** 8.02 (1H, d, J= 8.4 Hz); 7.78-7.54 (3H, m); 7.69 (1H, s); 7.58 (1H, d, J= 8.4 Hz); 7.26 (2H; d, J= 8.0 Hz); 2.34 (3H, s).

### Example 3: Synthesis of 3-(4-hydroxybut-1-inyl)-1-tosyl-1H-indole-5-carbonitrile (IV)

3-iodo-1-tosyl-1H-indole-5-carbonitrile **(V)** (12.5 g, 28.3 mmol), PdCl₂ (150 mg, 0.85 mmol), PPh₃ (668 mg, 2.55 mmol) and CuI (162 mg, 0.85 mmol) are suspended in a mixture of triethylamine (65 mL) and DMF (60 mL) under inert atmosphere. The mixture is heated to the temperature of 30°C, then treated with a solution (10 mL) obtained by dissolving 3-butyn-1-ol (2.7 mL, 30.0 mmol) in DMF added by slow dripping. At the end of the addition the reaction mixture is left to stand at ambient temperature and maintained under stirring overnight. The mixture is then diluted with ethyl acetate (200 mL) and treated with a solution of 1M HCl until markedly acid. The phases are separated and the organic phase is washed sequentially with a saturated solution of NaHCO₃ with the addition of a 33% solution of NH₄OH, an 0.1M solution of Na₂S₂O₃ and a saturated solution of NaCl. The organic phase is dried on Na₂SO₄, filtered, and concentrated at low pressure. Crude product **(IV)** is obtained as a solid (11 g), which is not purified but used "as is" in the subsequent reaction. A portion of the crude product is purified by flash chromatography (petroleum ether/AcOEt 50/50) to obtain chemically pure product (IV) as a white solid.

**¹H-NMR (400 MHz, CDCl₃), δ:** 7.97 (1H, d, J= 8.4 Hz); 7.89 (1H, s); 7.71 - 7.68 (3H, m); 7.50 (1H, d, J= 8.8 Hz); 7.20 (1H, d, J= 8.4 Hz); 3.78 (2H, m); 3.65 (1H, bs); 2.67 (2H, t, J= 6.4 Hz); 2.29 (3H, s).

### Example 4: 3-(4-hydroxybutyl)-1-tosyl-1H-indole-5-carbonitrile (II)

Crude 3-(4-hydroxybutyn-1-yl)-1-tosyl-1H-indole-5-carbonitrile **(IV),** prepared as described in Example 3 (1.42 g, 3.90 mmol), dissolved in MeOH (30 mL), is treated with Pd(OH)₂/C 20% w/w (281 mg). The system is rendered inert and left under stirring overnight under H₂ atmosphere at ambient pressure. The end-of-reaction mixture is then filtered through Celite^{®}, and the solution is concentrated at low pressure. The crude product is purified by flash chromatography (petroleum ether/AcOEt: 50/50) to obtain the chemically pure compound of formula **(II)** as a solid (1.48 g) with a yield of 96%.

**¹H-NMR (400 MHz, CDCl₃), δ:** 7.99 (1H, d, J= 8.4 Hz); 7.76 (1H, s); 7.78 (2H, d, J= 8.8 Hz); 7.47 (2H, d, J= 8.4 Hz); 7.40 (1H, s); 7.19 (2H, d, J= 8.4 Hz); 3.50 (2H, m), 2.63 (2H, t, J= 7 Hz); 1.73-1.68 (2H, m); 1.61-1.56 (2H, m).

### Example 5: Synthesis of 4-(5-cyano-1-tosyl-1H-indol-3-yl)butyl methanesulphonate (II)

3-(4-hydroxybutyl)-1-tosyl-1H-indole-5-carbonitrile **(II)** (486 mg, 1.32 mmol), prepared as described in Example 4, is dissolved in CH₂Cl₂ (15 mL) in the presence of Et₃N (277 µL, 1.98 mmol) under inert atmosphere. The solution is cooled to 0°C and treated with mesyl chloride (156 µL, 1.58 mmol). The reaction mixture is maintained under stirring for 1 h at ambient temperature and then treated sequentially with a solution of 1M HCl, saturated NaHCO₃, and finally with a saturated solution of NaCl. The organic phase is dried on Na₂SO₄, concentrated at low pressure and the residue purified by flash chromatography (petroleum ether/AcOEt 50/50). Product **(II)** (519 mg, 1.16 mmol) is obtained as a solid with a yield of 88%.

**¹H-NMR (400 MHz, CDCl₃), δ:** 8.01 (1H, d, J= 8.6); 7.77 (1H, s); 7.71 (2H, d, J= 8.2 Hz); 7.51 (1H, d, J= 8.6 Hz); 7.42 (1H, s); 7.21 (2H, d, J= 8.2 Hz); 4.23 (2H, t, J= 5.2 Hz); 2.98 (3H, s); 2.67 (2H, m); 2.31 (3H, s); 1.78-1.77 (2H, m).

### Example 6: Synthesis of ethyl 5-(4-(4-(5-cyano-1-tosyl-1H-indol-3-yl)butyl)piperazin-1-yl)benzofuran-2-carboxylate (IX), with P= tosyl and W= -OEt

4-(5-cyano-1-tosyl-1H-indol-3-yl)butyl methanesulphonate **(II)** (100 mg, 0.22 mmol), prepared as described in Example 5, and ethyl 5-(piperazin-1-yl)benzofuran-2-carboxylate of formula **(VIII)** (62 mg, 0.22 mmol), are dissolved in CH₃CN (4 mL) and treated with K₂CO₃ (91 mg, 0.66 mmol) under inert atmosphere. The reaction mixture is maintained under stirring at reflux temperature overnight, and then diluted with AcOEt and H₂O. The phases are separated and the organic phase is dried on Na₂SO₄, concentrated at low pressure, and the residue is purified by flash chromatography (petroleum ether/AcOEt 50/50). Chemically pure ethyl 5-(4-(4-(5-cyano-1-tosyl-1H-indol-3-yl)butyl)piperazin-1-yl)benzofuran-2-carboxylate (97 mg, 0.16 mmol) of formula **(IX)** is obtained with a yield of 72%.

**¹H-NMR (400 MHz, CDCl₃), δ:** 8.01 (1H, d, J= 8.4 Hz); 7.78 (1H, s); 7.71 (2H, d, J= 8.4 Hz); 7.51 (1H, d, J= 8.2 Hz); 7,44-7.40 (3H, m); 7.21 (2H, d, J= 8.4 Hz); 7.13 (1H, dd, J=9.2, 2.4 Hz); 7.07 (1H, s); 4.39 (2H, q, J= 7.2 Hz); 3.15 (4H, t, J= 4.8 Hz); 2.66 (2H, t, J= 7.4 Hz); 2.59 (4H, t, J= 4.8 Hz); 2.41 (2H, t, J= 7.4 Hz); 2.31 (3H, s); 1.74-1.66 (1H, m); 1.61-1.56 (1H, m); 1.38 (3H, t, J= 7.2 Hz).

### Example 7: Synthesis of a compound of formula (IV), wherein X is OH and P is Boc (internal code: VILA-D Boc)

In a 500 ml flask, 50 g (155.7 mmol) of a compound of formula **(V)** wherein Y is Br and P is Boc are dissolved in 60 ml of THF and 50 ml of triethylamine (TEA) at 60°C. The solution is outgassed with nitrogen for 20 minutes.

593 mg (3.11 mmol) of CuI and 1.63 g (6.23 mmol) of Ph₃P are added; after 15 minutes 276 mg (1.56 mmol) of PdCl₂ are added and the mixture left to mix for other 15 minutes.

12 g (171.3 mmol) of 3-butyn-1-ol are dissolved in 12 ml of THF are dripped in within 4 hours, keeping the temperature at 60°C.

After one hour and 30 minutes from the addition of 3-butyn-1-ol, the reaction mixture is cooled to 40°C and 150 ml of toluene and 150 ml of water are added. The mixture is filtered on a perlite panel and the phases are separated.

The organic phase is washed four times with 75 ml of an aqueous solution of 5% ammonia at 40°C.

The solvent is distilled off from the organic phase, till a residue is obtained. The residue is suspended in 48 ml of toluene at 50-55°C for 45 minutes. The suspension is cooled to 0-5°C and filtered. The panel is washed with 40 ml of toluene cooled at 0°C.

45.13 g of wet product are obtained, which is dried under vacuum at a temperature of 50°C overnight.

41.97 g of dry product are obtained. Yield = 86.9%

**¹H-NMR (300 MHz, CDCl₃),** δ: 8.23, d (J=9 Hz), 1H; 7.98, s, 1H; 7.79, s, 1H; 7.58, d (J=9 Hz), 1H; 3.87, m, 2H; 2.77, m, 3H; 1.67, s, 9H.

### Example 8: Synthesis of a compound of formula (IV), wherein X is OH and P is Boc (internal code VILA-D Boc)

In a 500 ml flask, 50 g (155.7 mmol) of a compound of formula (V), wherein Y is Br and P is Boc, are dissolved in 50 ml of dimethylacetamide (DMA) and 150 ml of triethylamine(TEA) at 60°C. The solution is outgassed with nitrogen for 20 minutes.

593 mg (3.11 mmol) of CuI and 1.63 g (6.23 mmol) of Ph₃P are added. After 15 minutes 276 mg (1.56 mmol) of PdCl₂ are added and the mixture left for 15 minutes.

12 g (171.3 mmol) of 3-butyn-1-ol dissolved in 12 ml of THF are dripped in within 4 hours, keeping the temperature at 60°C.

After 1 hour and 30 minutes from the addition of 3-butyn-1-ol, the reaction mixture is cooled to 30-35°C and 150 ml of ethyl acetate and 150 ml of water are added. The mixture is filtered on a perlite panel and the phases are separated. 270 g of 10% HCl are dripped in keeping the temperature at 25-30°C and the phases are separated.

100 ml of ethyl acetate are added to the organic phase and it is washed four times with 75 ml of an aqueous solution containing 5% ammonia and once with 75 ml of an aqueous solution containing 10% NaCl at 30-35°C.

The solvent is distilled from the organic phase under vacuum to obtain a residue, which is diluted with 25 ml of ethyl acetate at a temperature of 50-55°C for 20 minutes.

The suspension is cooled to 0-5°C and filtered. The panel is washed with 25 ml of ethyl acetate cooled at 0-5°C.

48 g of wet product are obtained that is dried under vacuum at 50°C overnight.

38.5 g of dried product are obtained. Yield = 79.7%

**¹H-NMR (300 MHz, CDCl₃),** δ: 8.23, d (J= 9 Hz), 1H; 7.98, s, 1H; 7.79, s, 1H; 7.58, d (J= 9 Hz), 1H; 3.87, m, 2H; 2.77, m, 3H; 1.67, s, 9H.

### Example 9: Synthesis of a compound of formula (II) wherein X is OH and P is Boc (internal code: VILA-E Boc)

38.5 g (124 mmol) of a compound of formula **(IV),** wherein X is OH and P is Boc, dissolved in 250 ml of THF and 2.64 g (0.62 mmol) of Pd/C 5% w/w are loaded in autoclave. The mixture is kept under vigorous stirring, and hydrogen, at a pressure of 4 atm, is loaded.

After 2 hours the reaction mixture is filtered on a perlite panel and the panel is washed with THF.

The solvent is distilled under vacuum so obtaining 42 g of an oily residue. Yield = 98%.

**¹H-NMR (300 MHz, CDCl₃), δ:** 8.21, d (J= 8.4 Hz), 1H; 7.84, s, 1H; 7.50, d (J= 8.7 Hz), 1H; 7.45, s, 1H; 3.70, t (J= 6.3 Hz), 2H; 2.72, t (J= 6.9 Hz), 2H; 1.79, m, 4H; 1.67, s, 9H

### Example 10: Synthesis of a compound of formula (II) wherein X is OSO₂CH₃ and P is Boc (Internal code: VILA-F Boc)

74.5 g (237 mmol) of a compound of formula **(II),** wherein X is OH and P is Boc, are dissolved in 370 ml of THF.

27.6 g (273 mmol) of triethylamine are added and the solution is cooled to -5°C.

29.9 g (261 mmol) of methansulfonyl chloride are dripped in, maintaining the temperature of the reaction mixture under 5°C.

The mixture is maintained in these conditions for one hour, after that 75 ml of water are added, keeping the temperature under 5°C.

The reaction mixture is heated at 20-25°C and filtered on a perlite panel. The panel is washed with THF. 150 ml of toluene are added and the phases are separated. The organic phase is washed with 75 ml of water and then with 75 ml of an aqueous solution of 10% NaCl.

The solvent is distilled off under vacuum obtaining an oily residue of 98 g. Yield = 98%.

**¹H-NMR (300 MHz, CDCl3), δ:** 8.22, d (J= 8.7 Hz), 1H; 7.84, s, 1H; 7.56, dd (J= 9 Hz, 1.8 Hz), 1H; 7.47, s, 1H; 4.28, t (J= 5.4 Hz), 2H; 3.01, s, 3H; 2.74, t (J= 6 Hz), 2H; 1.85, m, 4H; 1.68, s, 9H.

## Claims

1. A process for the preparation of a compound of formula **(II)** 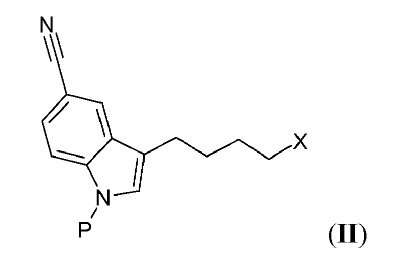 wherein X is -OH or a leaving group, and P is H or a protecting group of the indole nitrogen, comprising reducing a compound of formula **(III)** or a compound of formula **(IV)** 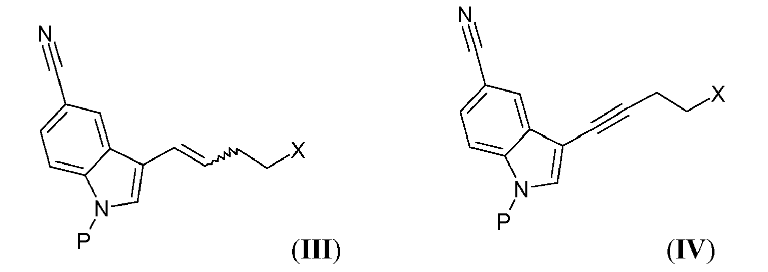 wherein X and P are as defined above and, if desired, converting a compound of formula **(II)** into another compound of formula **(II).**

2. A process according to claim 1, wherein in a compound of formula **(IV)** and in a compound of formula **(III)** being X and P as defined above, X is other than O-CH₂-phenyl and P is other than tosyl.

3. A process according to claim 1, wherein a compound of formula **(III)** or formula **(IV)** is reduced by catalytic hydrogenation in the presence of a homogeneous or heterogeneous metal catalyst, typically a Pd, Pt, Ni, Rh or Ru- based catalyst, preferably a Pd-based catalyst, at a hydrogen pressure ranging between about 1 atm and 10 atm.

4. A process according to claim 3, wherein the concentration of the metal on the support ranges from about 1 to 30%, preferably between about 5 to 20%.

5. A process according to claim 3 or 4, wherein the molar ratio of the catalyst to the compound of formula **(II)** ranges between about 0.1 and 10%, preferably between about 0.5 and 5%.

6. A process according to claims 1-5, wherein the reduction is performed in the presence of an organic solvent selected from an aprotic polar solvent; a cyclic or acyclic ether; a chlorinated solvent; an apolar aprotic solvent; a polar protic solvent, such as a straight or branched C₁-C₆ alkanol or water; an ester; a straight or branched C₃-C₇ ketone; a carboxylic acid; or a mixture of two or more of said solvents; or in a solution of a mineral acid, such as hydrochloric or sulphuric acid, or a mixture thereof with one, two or three of the above defined organic solvents; the reduction is preferably performed in methanol or tetrahydrofuran.

7. A process according to claim 1 or 2, wherein the reduction of a compound of formula **(III)** or formula **(IV)** is performed by transfer hydrogenation, using a homogeneous or heterogeneous metal catalyst, as defined in claims 3 and 4.

8. A process according to claim 1 to 7, wherein the reduction of a compound of formula **(III)** or **(IV)** is carried out at a temperature ranging between about 0°C and the reflux temperature of the solvent, preferably between about 0°C and ambient temperature.

9. A process according to claims 3-8, wherein the hydrogen pressure is atmospheric pressure.

10. A compound of formula **(III)** or **(IV)** 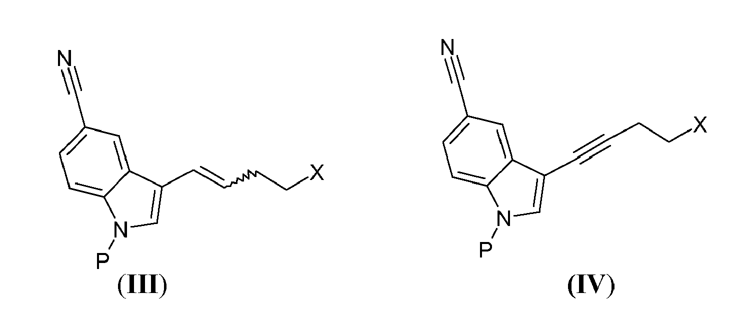 wherein X is -OH or a leaving group, and P is H or a protecting group of the indole nitrogen and, in a compound of formula **(III),** the double bond -C=Con the aliphatic chain has either (E) or (Z) stereochemistry, or a mixture thereof; and wherein when in a compound of formula **(III)** P is tosyl, X is other than OCH₂Ph.

11. A compound of formula **(IV)** according to claim 10, wherein P is Boc or tosyl and X is OH or a methanesulphonyl group.

12. Use of a compound of formula **(III)** or **(IV)** 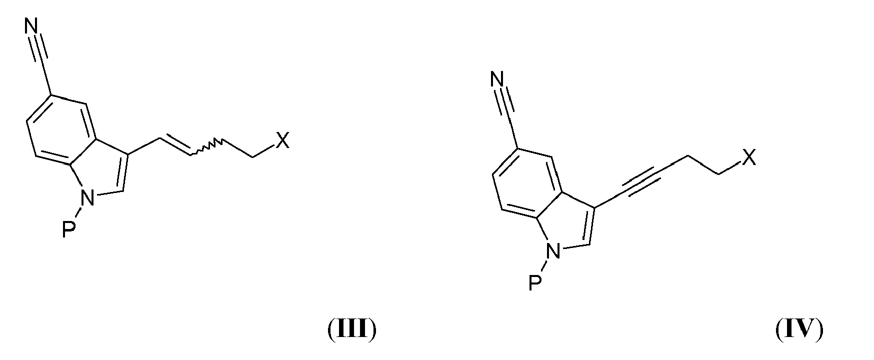 wherein X is OH or a leaving group and P is H or a protecting group of the indole nitrogen and in a compound of formula **(III)** the double bond -C=Con the aliphatic chain has (E) or (Z) stereochemistry, or a mixture thereof, as intermediate in the preparation of Vilazodone of formula **(I)** 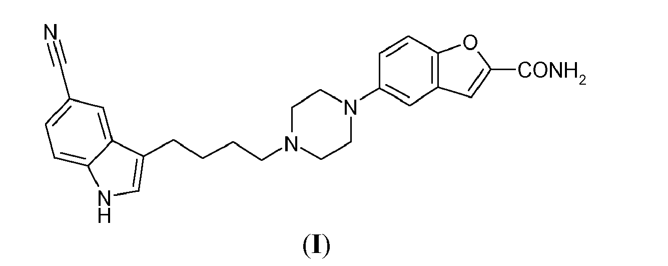 or a salt thereof.

13. A process according to claims 1-9, further comprising alkylating a compound of formula **(VIII),** or a salt thereof, 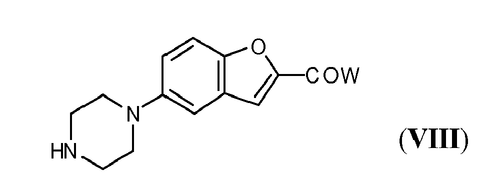 wherein W is -OH or -OR₁ group, wherein R₁ is a straight or branched C₁-C₆ alkyl, or -NH₂, with a compound of formula **(II),** wherein X is a leaving group and P is H or a protecting group of the indole nitrogen, to obtain a compound of formula **(IX),** or a salt thereof, 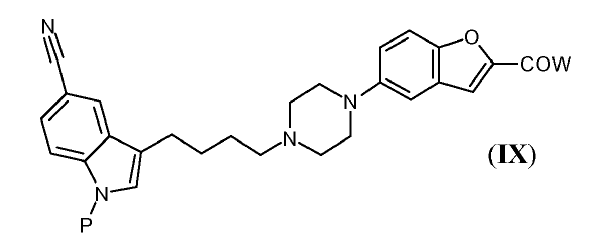 wherein W is as defined above and P is H or a protecting group of the indole nitrogen and, if applicable, converting a compound of formula (IX) into another compound of formula (IX), and/or removing the protecting group to obtain a compound of formula (I), or a salt thereof

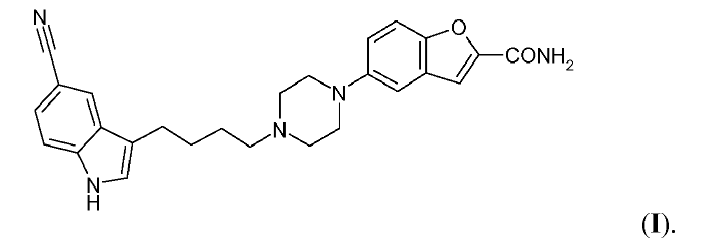

14. A process according to claim 1, further comprising converting a compound of formula **(II)** into a compound of formula **(IX)** or a salt thereof 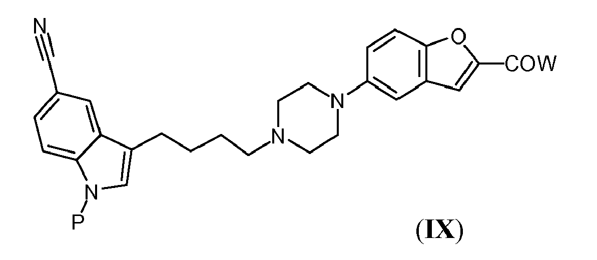 wherein W is an -OH or -OR₁ group, wherein R₁ is a straight or branched C₁-C₆ alkyl group, or -NH₂ and P is H or a protecting group of the indole nitrogen; or converting a compound of formula **(II)** into a compound of formula **(I)** or a salt thereof.

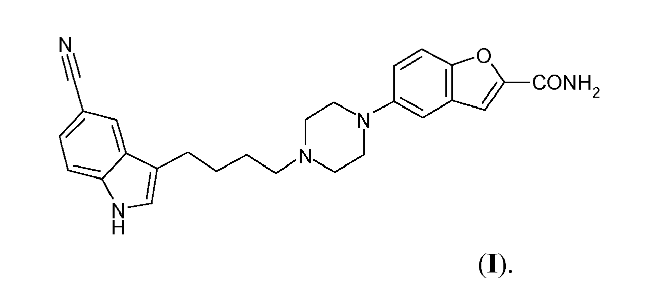

15. A process for the preparation of a compound of formula (IX) or a salt thereof 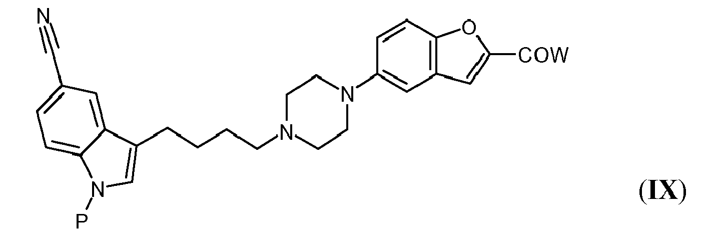 wherein W is an -OH or -OR₁ group, wherein R₁ is a straight or branched C₁-C₆ alkyl group, or -NH₂ and P is H or a protecting group of the indole nitrogen; or for the preparation of a compound of formula **(I)** or a salt thereof 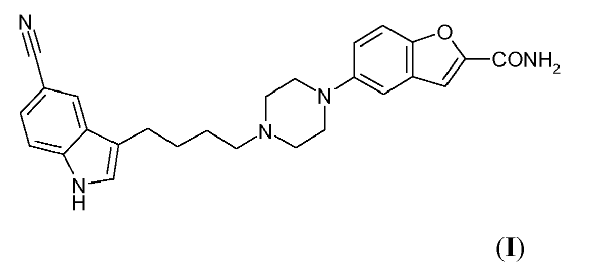 comprising using a compound of formula **(III)** or **(IV),** as defined in claim 12, as starting material.
